# EUROPÄISCHE PATENTSCHRIFT

(11) **EP 1 469 866 B1**
(45) Veröffentlichungstag und Bekanntmachung des Hinweises auf die Patenterteilung: **13.05.2009**
(21) Anmeldenummer: 03706365.8
(22) Anmeldetag: 20.01.2003
(51) Int. Cl.: A61K 31/715, A61P 31/04

(54) **CYCLOGLYCANE GEEIGNET ZUR VERHINDERUNG DER INFEKTION VON SÄUGETIERZELLEN**
CYCLOGLYCANS SUITABLE TO INHIBIT MAMMALIAN INFECTION
CYCLOGLYCANES POUVANT INHIBER UNE INFECTION CHEZ LES MAMMIFERES

(30) Priorität: 01.02.2002 DE 10203999
(43) Veröffentlichungstag der Anmeldung: 27.10.2004
(73) Patentinhaber: N.V. Nutricia, 2712 HM Zoetermeer (NL)
(72) Erfinder: STAHL, Bernd, 61191 Rosbach (DE); FINKE, Berndt, 32049 Herford (DE); SCHMITT, Joachim, 63768 Hösbach (DE); GOEBEL, Werner, 97218 Gerbrunn (DE); SLAGHIUS, Jörg, 97082 Würzburg (DE); BOEHM, Günther, 61209 Echzell (DE)
(74) Vertreter: Köster, Hajo
(86) Internationale Anmeldenummer: PCT/EP2003/000505
(87) Internationale Veröffentlichungsnummer: WO 2003/063882

(56) Entgegenhaltungen:
- WO-A-90/00596
- US-A- 5 221 669
- US-A- 5 296 472
- US-B1- 6 261 540
- DATABASE WPI Section Ch, Week 200056 Derwent Publications Ltd., London, GB; Class B04, AN 1995-308963 XP002241408 & JP 03 101754 B (AMANO PHARM KK), 23. Oktober 2000 (2000-10-23)
- DATABASE WPI Section Ch, Week 199927 Derwent Publications Ltd., London, GB; Class A60, AN 1999-323408 XP002241409 & JP 11 116410 A (TAKEDA CHEM IND LTD), 27. April 1999 (1999-04-27)

## Beschreibung

Die Erfindung betrifft die Verwendung von insbesondere homopolymeren Cycloglycanen mit einer ringförmigen Grundstruktur aus 2 bis 40 Monosacchariden im Ring zur Verhinderung der Invasion und Infektion von Säugerzellen durch Pathogene und zur Bekämpfung von durch derartige Pathogene verursachten Erkrankungen.

Die US-A-5 221 669 beschreibt antivirale Mittel, welche α-Cyclodextrinsulfates alleine oder zusammen mit bekannten antiviralen Agenzien enthalten. Diese bekannten Mittel sollen zur Hemmung der Infektion des HIV-Virus wirksam sein.

Aus der WO 90 00596 A sind ebenfalls Cyclodextrine enthaltende antivirale Mittel zur Bekämpfung des HIV-Virus bekannt.

XP002241408 und JP 03 101754B befassen sich mit verzweigten Cyclodextrinen, die als Konservierungsmittel für unter anderem Nahrungsmittel eingesetzt werden.

Die Adhäsion sowohl pathogener Organismen als auch zellschädigender Substanzen an die Oberfläche von Säugerzellen ist der erste Schritt und eine unabdingbare Voraussetzung für eine Infektion bzw. Schädigung der Zelle. Die Interaktion zwischen den Pathogenen und den Zellen kommt durch eine Ligand-Rezeptor-Beziehung zustande. Glycostrukturen spielen bei diesen Beziehungen bzw. Wechselwirkungen eine wichtige Rolle.

Eine Möglichkeit, derartige Ligand-Rezeptor-Beziehungen zu beeinflussen, besteht in der Blockierung und/oder der strukturellen Veränderung der jeweiligen Rezeptoren auf der Zelloberfläche oder der Liganden.

Verschiedene Kohlenhydratmischungen haben sich in spezifischen Testsystemen als sehr wirksam erwiesen, die Adhäsion beispielsweise von Mikroorganismen an die Zelloberfläche zu vermindern oder ganz zu verhindern, man vergleiche: Kunz, C.; Rudloff, S. Acta Paediatr. 1993, 82, 903-912. Andere Substanzen wie etwa die Lewis-Strukturen als Kohlenhydrat-Liganden der Selektine (Adhäsionsproteine auf Endothelien und Lymphocyten), modulieren die Interaktion der Lymphocyten mit dem Endothel beispielsweise im Rahmen von Rolling, Homing und der Invasion bei entzündlichen Prozessen (Norman, K.E.; Anderson, G.P.; Kolb, H.C.; Ley, K.; Ernst, B. Blood 1998, 91, 475-483). Eine weitere wichtige physiologische Rolle im Zusammenhang sowohl mit grundlegenden zellulären Funktionen als auch mit spezifischen Funktionen wie Zelladhäsion, Migration, Chemotaxis, Proliferation, Apoptose, Neuritenwachstum erfüllen die Galactose-erkennenden Lektine, die Galectine. (Cooper DN & Barondes SH, Glycobiology 1999 9 (10) 979-984). Für den Nematoden C. elegans konnte gezeigt werden, dass dessen Galectin LEC-1 verschiedene Galactose enthaltende Oligosaccharid-Derivate mit unterschiedlicher Spezifität binden kann. (Arata Y. Hirabayashi J. Kasai K, JBC, 2001:276, 5, 3068-3077). In einem Mäusemodell konnte durch den Einsatz von Galactose spezifischen Lectinen die Letalität einer experimentellen Listeriose verringert werden (Stoffel B, Beuth J, Pulverer G, Zentralbl. Bakteriol. 1996, 284:439-442). Die Adhärenz von Mikroorganismen an Wirtszellen kann aber auch der Auslöser von Signalkaskaden sowohl in den körperfremden pathogenen als auch in den körpereigenen Zellen sein.

Eine andere Möglichkeit besteht darin, auf molekularbiologischer Ebene eine Beeinflussung zellulärer Prozesse zu erreichen. Dies kann dazu führen, dass etwa bei den Säugerzellen Abwehrmechanismen ausgelöst werden oder bei den pathogenen Mikroorganismen die Expression von Virulenzmechanismen (z.B. Abschalten von Virulenzgenen bei Bakterien durch Blockierung zentraler Regulatoren) verringert oder verhindert werden. Auf diese Weise kann die Expression bestimmter Oberflächenstrukturen von pathogenen Listerien, die für die Invasion in Wirtszellen verantwortlich sein, durch bestimmte Kohlenhydrate wie Cellobiose erfolgreich gehemmt werden (Park SF Kroll RH Mol Microbiol 1993 8:653:661; WO-A 94/02586).

Aufgabe der vorliegenden Erfindung ist es, einen Weg aufzuzeigen, wie mit Hilfe von Kohlenhydraten die Invasion und Infektion von Säugerzellen durch Pathogene verringert oder verhindert werden kann und durch derartige Pathogene verursachte Erkrankungen wirksam bekämpft werden können.

Gelöst wird diese Aufgabe durch die Lehre der Ansprüche.

Erfindungsgemäß werden spezielle Cycloglycane zur Lösung der erfindungsgemäßen Aufgabe zur Anwendung gebracht. Diese Cycloglycane werden nachstehend als erfindungsgemäße Cycloglycane bezeichnet.

Erfindungsgemäß können sowohl einzelne erfindungsgemäße Cycloglycane alleine oder mehrere erfindungsgemäße Cycloglycanen in Mischung miteinander eingesetzt werden. Ferner ist es möglich, ein erfindungsgemäßes Cycloglycan oder mehrere erfindungsgemäße Cycloglycane oder auch eine Mischung von zahlreichen erfindungsgemäßen Cycloglycanen zusammen mit anderen, nicht zu den erfindungsgemäßen Cycloglycanen zählenden Kohlenhydraten in Form einer Kohlenhydratmischung einzusetzen.

Die erfindungsgemäßen Cycloglycane weisen im Ring 2 bis 40 Monosaccharide auf; an sich handelt es sich dabei um mit einander verbundene Monosaccharid-Einheiten, die der besseren Darstellung halber hier nur als Monosaccharide bezeichnet werden. Diese Monosaccharide stellen ein Ringpolymer dar. Die erfindungsgemäßen Cycloglycane können somit 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 35, 36, 37, 38, 39 und 40 Monosaccharide im Ring besitzen.

Vorzugsweise sind die erfindungsgemäßen Cycloglycane aus 6 bis 40, insbesondere bevorzugt aus 6 bis 20 und weiterhin insbesondere bevorzugt aus 6 bis 8 Monosacchariden aufgebaut.

Weiterhin bevorzugt handelt es sich bei den erfindungsgemäßen Cycloglycanen um homopolymere Cycloglycane. Mit anderen Worten, die ringförmige Grundstruktur weist nur Monosaccharide einer Art auf bzw. ist aus denselben Monosacchariden aufgebaut.

Der Ring der Cycloglycane ist vorzugsweise aufgebaut aus D-Fructose, D-Mannose, L-Fucose, D-N-Acetylglucosamin, D-N-Acetylgalactosamin, D-Xylose, Sialinsäuren (z. B. N-Acetylneuraminsäure), L-Rhamnose, D-Arabinose, D-Allose, D-Talose, L-Idose, D-Ribose, D-Galacturonsäure, Altrose, D-Galactose und Glucosen. Diese Monosaccharide sind ringförmig miteinander verbunden und stellen den Ring der erfindungsgemäßen, insbesondere homopolymeren Cycloglycane dar.

Bei den Cycloglycanen können die glycosidischen Verknüpfungen sowohl im Ring als auch bei den mit dem Ring verknüpften, nachstehend näher beschriebenen Glycanen folgende sein: α1-2, α1-3, α1-4, α1-6, α2-3, α2-6, α2-8, β1-2, β1-3, β1-4, β1-6 und β2-1.

Die Bindung der Monosaccharide untereinander ist vorzugsweise α- oder β-glycosidisch. Bevorzugte erfindungsgemäße Cycloglycane sind solche, die 6, 7 oder 8 Glucoseeinheiten im Ring in α-1-4-glycosidischer Verknüpfung aufweisen. Dazu zählen die hier genannten α-, β- und γ-Cyclodextrine und die Derivate davon.

Bei den erfindungsgemäßen Cyclodextrinen kann es sich um underivatisierte Cyclodextrine handeln. Es wird daher davon ausgegangen, ohne an dieser Erklärung gebunden zu sein, dass der ringförmige Aufbau der erfindungsgemäßen Cycloglycane für deren im Rahmen der hier vorliegenden Unterlagen beschriebenen Wirkungen verantwortlich ist.

Die erfindungsgemäßen Cycloglycane können auch - wie gesagt - derivatisiert sein, und zwar durch eine oder mehrere funktionelle Gruppe(n). Die Substitution kann am Ring selbst oder an den freien Hydroxylgruppen der diesen Ring bildenden Monosaccharide vorhanden sein.

Bevorzugte derivatisierte Cycloglycane sind solche, bei denen eines oder alle der folgenden Merkmale erfüllt sind:
i) eine oder mehrere der OH-Gruppen eines oder mehrerer der den Ring bildenden Monosaccharide ist bzw. sind durch eine NH₂-, SH-, Phosphat-Sulfat-, Nitrat-, Alkyl-, Hydroxyalkyl- oder Carboxyalkyl-Gruppe ersetzt. Bei den Alkyl-, Carboxyalkyl- und Hydroxyalkyl-Gruppen weist der Alkylrest vorzugsweise 1 bis 6 Kohlenstoffatome auf. Die den Ring bildenden Monosaccharide können somit beispielsweise durch eine oder mehrere Methyl-, Ethyl-, Hydroxyethyl-, Carboxymethyl- und Hydroxypropyl-Gruppen, um nur einige zu nennen, derivatisiert sein.
ii) eine oder mehrere der OH-Gruppen sowie der eventuell vorhandenen NH₂- und SH-Gruppen der den Ring bildenden Monosaccharide sind in Form von Ethern, Estern, Amiden und Iminen derivatisiert. Bei diesen Derivaten kann es sich beispielsweise um Succinyl-, Acyl- (insbesondere mit 1 bis 25 C-Atomen, weiterhin insbesondere mit 1, 2, 3, 4, 5, oder 6 C-Atomen, z. B. Acetyl-) Methylmalonsäureester-, Phosphoglyceryl- und Phosphocholinyl-Derivate handeln. Zu den Acylderivaten zählen ferner insbesondere solche mit ernährungsphysiologisch wertvollen gesättigten und ungesättigten Fettsäuren mit 12 bis 22 C-Atomen

Weiterhin bevorzugte erfindungsgemäße Cycloglycane sind folgende:
Hydroxypropyl-Cyclodextrine, Cyclofructine, Cyclomannine, Cyclogalactine und Cycloaltrine.

Bei den erfindungsgemäßen Cycloglycanen handelt es sich um bekannte Verbindungen oder sie können nach bekannten Verfahren hergestellt werden. Cycloglycane aus kleinen Ringen (beispielsweise mit nur 2 Monosacchariden im Ring) sind beschrieben in: Armspach D., Gattuso G., Königer R., Stoddart JF Cyclodextrins in: Bioorganic Chemistry: Carbohydrates. (SM Hecht ed.) Oxford Univ. Press New York 1999.

Zu den erfindungsgemäßen Cycloglycanen zählen die Cyclodextrine, bei denen der Ring aus dem Monosaccharid bzw. der Monosaccharideinheit Glucose aufgebaut ist. Sie kommen in natürlicher Weise als α-, β- und γ-Cyclodextrin vor. Diese Cyclodextrine werden bevorzugt erfindungsgemäß eingesetzt. Sie werden enzymatisch aus Stärke durch die Aktivität von Cyclodextrin Glycosyltransferasen (CGTasen), einem mikrobiellen Enzym (z. B. Bacillus macerans), gewonnen. Die Unterscheidung in die drei natürlichen Cyclodextrine basiert auf der Anzahl der beteiligten Glucose-Moleküle. α-Cyclodextrin beinhaltet 6, β-Cyclodextrin beinhaltet 7 und γ-Cyclodextrin beinhaltet 8 Glycopyranoseeinheiten, die jeweils in α-1-4-glycosydischen Bindungen zu einem Ring verknüpft sind. Weitere Cyclodextrine mit größeren Molekülen mit üblicherweise bis zu 10 Monosaccharideinheiten sind beilspielsweise beschrieben in MJ Playne & R. Crittenden, Commercial available oligosaccharides, Bulletin of the IDF 313, 1996, 10-22. Andere Cycloglycane, bevorzugte β-1-2-verknüpfte Cycloglycane, werden im periplasmatischen Raum verschiedener Bakterien nachgewiesen. Ringförmige Moleküle bestehend aus bis zu 40 Monosaccharideinheiten, die derivatisiert (insbesondere an den freien Hydroxylgruppen) sein können, sind z.B. beschrieben in: man vergleiche Talaga P., Stahl B., Wieruszeski J.-M., Hillenkamp F., Tsuyumu S., Lippens G., Bohin J.-P., Cell associated Glucans of Burkholderia solanacearum and Xanthomonas Campestris pv. Citri: a New Family of Periplasmic Glucans; Journal of Bacteriology 1996, 178, 8, 2263-2271.

Die erfindungsgemäßen Cycloglycane können nach geeigneten, bekannten Verfahren chemisch, enzymatisch oder in einer Kopplung beider Technologien hergestellt. Diese erfolgt systematisch aus den Monosaccharidbestandteilen oder durch Modifizierung geeigneter Oligosaccharid-Rohstoffe. Bei den enzymatischen Synthesen werden sowohl Transferasen (Leloir, bzw. nicht-Leloir) als auch Hydrolasen (reverse Hydrolyse bzw. Transglycosylierung) eingesetzt. Die Enzyme können dabei sowohl frei als auch eingebunden (etwa Membranreaktor) oder kovalent an einen Träger (beispielsweise beads, Chromatographiematerial oder Filtrationsmembranen) gebunden sein. Es ist auch möglich, pro- oder eukaryontische Zellen zur Synthese einzusetzen, sofern diese Zellen die geeigneten Enzyme aufweisen. Bezüglich weiterer Einzelheiten der Herstellung der erfindungsgemäßen Cycloglycane wird beispielsweise verwiesen auf Carbohydrates in Chemistry and Biology (Herausgeber Ernst, Hart, Sinay) Wiley VCH-Weinheim 2000 Vol. I-IV).

Weitere erfindungsgemäße Cycloglycane sind an folgenden Literaturstellen beschrieben oder können nach den dort beschriebenen Verfahren hergestellt werden: Armspach D., Gattuso G., Königer R., Stoddart JF Cyclodextrins in: Bioorganic Chemistry: Carbohydrates (SM Hecht ed.) p. 458, Oxford Univ. Press New York 1999.

Robyt JF Cyclodextrins in: Essentials of Carbohydrate Chemistry (CR Cantor ed.) Springer, New York, 1998.

Ergänzend wird auf folgendes hingewiesen. Ist das erfindungsgemäße Cycloglycan nur aus 2 Monosacchariden aufgebaut, dann ist es nicht substituiert; vielmehr besteht es nur aus dem Ring aus diesen 2 Monosacchariden.

Es wurde nun überraschend gefunden, dass die erfindungsgemäßen Cycloglycane die Invasion und Infektion von Säugerzellen durch Pathogene zumindest verringern oder sogar verhindern und zur Bekämpfung von durch derartige Pathogene verursachte Erkrankungen eingesetzt werden können. Zu diesen Pathogenen zählen invasive gram-positive und gram-negative, pathogene Bakterien, beispielsweise intrazelluläre Bakterien, insbesondere Listerien, und pathogene Viren, beispielsweise Rotaviren.

Es wurde beispielsweise gefunden, dass die erfindungsgemäßen Cycloglycane die Invasion und Infektion von Säugerzellen durch Listerien, insbesondere Listeria monocytogenes verhindern können. Die Ergebnisse der durchgeführten Untersuchungen zeigen deutlich, dass weder der Vorgang der Phagozytose an sich, noch die Replikation der aufgenommenen Listerien gehemmt wird. Als besonders stark inhibitorisch erwies sich unter anderem die erfindungsgemäßen Cyclodextrine.

Insgesamt konnte festgestellt werden, dass die erfindungsgemäßen Cycloglycane eine antiinfektive bzw. inhibitorische Wirkung im Sinne einer Infektion mit Listerien und Salmonellen besitzen. Die erfindungsgemäßen Cycloglycane können die Invasion von Listerien in Makrophagen-Zelllinien verhindern. Da der Infektion einer Zelle häufig eine Adhärenz und eine Invasion der Pathogene vorausgeht, ist eine Übertragbarkeit auf alle Pathogene gegeben, deren Infektion nach diesem Mechanismus der Listerien verläuft. Es sind dies vor allem Salmonellen und E. coli.

Bisher war von den erfindungsgemäßen Cycloglycanen nur bekannt, dass sie andere Substanzen und Substanzklassen komplexieren können, um deren Löslichkeitsverhalten zu verbessern. Erfindungsgemäß konnte jedoch festgestellt werden, dass die Cycloglycane anti-infektive Eigenschaften besitzen, wodurch diese Verbindungen zur Verhinderung oder Therapie einer Infektion eingesetzt werden können, ohne dass ein Zusatz weiterer Substanzen erforderlich ist. Erforderlichenfalls können natürlich auch weitere Wirksubstanzen zusammen mit den erfindungsgemäßen Cycloglycanen zur Anwendung gelangen.

Die erfindungsgemäßen Cycloglycane können nicht nur als freie bzw. ungebundene Cyclooligosaccharide sondern auch an einen Träger gebunden oder daran immobilisiert, z. B daran adsorbiert, eingesetzt werden. Bei diesem Träger kann es sich um ein Peptid/Protein (beispielsweise BSA), ein Lipid, (Glycolipid, Ceramid), ein Polymer oder ein Biopolymer (beispielsweise Kohlenhydrat-Dendrimer, Polysaccharid, Polyacrylamid) oder jedes andere Aglykon handeln.

Die erfindungsgemäßen Cycloglycane, seien es nun ungebundene bzw. freie Cycloglycane oder an einen Träger gebundene Cycloglycane, können verschiedenen diätetischen Mitteln und pharmazeutischen Mitteln einverleibt sein. Alle diese Mittel können in der für die gewünschte Verabreichung geeigneten Form und insbesondere in flüssiger oder fester Form vorliegen. Zu diesen Mitteln zählen auch Nahrungsergänzungsmittel, Getränke sowie Nahrungen einschließlich Säuglings- und Babynahrungen. Der Begriff Babynahrung bzw. Säuglingsnahrung bezeichnet insbesondere alle künstlich hergestellten Nahrungen. Als "künstlich" werden hier solche Nahrungen verstanden, die aus Rohstoffen pflanzlichen und tierischen Ursprungs, jedoch nicht humanen Ursprungs, hergestellt werden. Diese Nahrungen können auf beliebige Weise an einen Menschen oder ein Tier verabreicht werden. Dazu zählt auch die Verabreichung als Infusionslösung und als Sondennahrung in den Magen. Die erfindungsgemäßen Cycloglycane können jedoch auch natürlichen Milchen, beispielsweise Tiermilchen hinzugefügt werden.

Die erfindungsgemäßen Cycloglycane können beispielsweise als Beimengungen oder Additive folgenden Produkten zugegeben werden, wobei diese Aufzählung nicht abschließend ist: Milch und Milchprodukte, Säuglings- und Kindernahrungen, Schokoriegel, Joghurtgetränke, Käse-, Wurst- und Fleischwaren, Aufbaunahrung, Sondennahrung und Produkten für Schwangere und zur Immunsuppression.

Neben den erfindungsgemäßen Cycloglycanen können in den erfindungsgemäßen Mittel auch weitere Kohlenhydrate vorhanden sein, so dass die erfindungsgemäßen Mittel ein Kohlenhydratmischung aufweisen, wobei die erfindungsgemäßen Cycloglycane ein Teil dieser Kohlenhydratmischung darstellen.

Die erfindungsgemäßen Cycloglycane können auch in Form eines pharmazeutischen Mittels alleine oder zusammen mit einem oder mehreren zusätzlichen Wirkstoff(en) verabreicht werden. Diese Mittel können beispielsweise als Tablette/Sachet formuliert sein. Für die Formulierung derartiger Pharmazeutika können übliche Adjuvantien, Träger, Hilfsmittel Verdünnungsmittel, feucht-haltende Mitteln, Verdickungsmittel, Geschmacksstoffe, Süßungsmittel usw. Anwendung finden.

Die pharmazeutischen Mittel können auf jede übliche Weise an einen Patienten (d.h. Mensch und Tier) verabreicht werden. Zweckmäßigerweise handelt es sich jedoch um Mittel, die zur oralen, lingualen, nasalen, intestinalen, bronchialen, vaginalen, topischen (Haut und Schleimhaut) und per os Verabreichung geeignet und entsprechend der Verabreichungsart formuliert sind.

Die mindestens ein erfindungsgemäßes Cycloglycan enthaltenden diätetischen Mittel und pharmazeutischen Mittel können unter anderem zur Prävention und Behandlung von Infektionen des Gastrointestinaltraktes, beispielsweise bei Listeriosen, des Blutsystems, der Atemwege, des Urogenitaltraktes sowie des Nasen-Rachenraumes und zum Schutz von Endothelien, Epithelien und Mukosa eingesetzt werden. Sie können somit auch topisch auf die Haut aufgetragen oder auch auf Schleimhäuten Anwendung finden. Zu diesen Schleimhäuten zählen nasale, intestinale, bronchiale und vaginale Schleimhäute. So können die erfindungsgemäßen Cycloglycane beispielsweise einem Mundspülmittel beigefügt sein. Als Zielgruppen für die erfindungsgemäßen Cycloglycane können alle Altersstufen von Neugeborenen bis zu Senioren genannt werden. Besondere Einsatzgebiete sind der Schutz und die Behandlung von Schwangeren, Kranken, geschwächten und älteren Menschen, bei denen die Vermeidung beispielsweise einer Listeriose von besonderer Bedeutung ist.

Nachstehend sind beispielhafte Diätetika und Pharmazeutika aufgeführt, welche mindestens ein erfindungsgemäßes Cycloglycan enthalten. Es handelt sich dabei um folgende erfindungsgemäße Cycloglycane: α-Cyclodextrin, β-Cyclodextrin, γ-Cyclodextrin, Cyclofructine (DP6-8), Cyclomannine (DP6-8), Cyclogalactine (DP6-8), Cycloaltrine (DP6-8), periplasmatische Cycloglucane (DP6-25). Der Einfachheit halber werden diese Cycloglycane in den Beispielen nur mit dem Begriff Cycloglycan bezeichnet. Dieser Begriff steht stellvertretend für jedes oben aufgeführte, erfindungsgemäße Cycloglycan und deren Mischungen.

### Beispiel 1:

Zur Herstellung von Sachets werden jeweils 100 mg Cycloglycan mit 990 mg Maltodextrin trocken gemischt und dann in Sachets verpackt. Diese Sachets werden dreimal täglich zu den Mahlzeiten verabreicht.

### Beispiel 2:

Eine bekannte Heilnahrung (d.h. Milupa® HN 25, bilanzierte Diät) in Form eines Perlates mit 18,8 g Eiweiß, 8,6 g Fett, 62,8 g Kohlenhydrate, 3,3 g Mineralstoffen und Vitaminen wird bei der per se bekannten Herstellung mit Cycloglycan in einer solchen Menge versetzt, dass 50 mg Cycloglycan in 100 g des fertigen Perlats enthalten sind.

Für die Herstellung einer flüssigen Heilnahrung werden 100 ml der bekannten Heilnahrung *Milupa HN 25 flüssig* (2,3 g Eiweiß, 1,6 g Fett, 8,5 g Kohlenhydrate, 37 g Mineralstoffe, Vitamine) mit 7 mg Cycloglycan versetzt.

### Beispiel 3:

### Produkt für Schwangere

Eine Brausetablette (Endgewicht 4,15 g) (*Neovin*® von Milupa) wird unter Beimengung von 200 bis 500 mg Cycloglycan auf per se bekannte Weise hergestellt. Täglich wird eine Tablette in 150 ml Wasser gelöst und getrunken.

### Beispiel 4:

### Produkt für Ältere und geschwächte Personen

Eine bilanzierte pulverförmige Aufbaunahrung (*Dilsana*® von Milupa) mit 22,5 g Eiweiß, 7,7 g Fett, 60,8 g Kohlenhydrate, 5,4 g Mineralstoffen und Vitaminen wird auf per se bekannte Weise unter Einarbeitung von 100 mg bis 1000 mg Cycloglycan pro 100 g Pulver hergestellt. Täglich werden bis zu 3 x 50 g der Nahrung in 150 ml Wasser gelöst und verabreicht.

### Beispiel 5

### Tee

100 g eines auf übliche Weise hergestellten Instant-Tee-Pulvers werden mit 2 g Cycloglycan vermengt. Je 3,8 g Teepulver werden in 100 ml heißem Wasser gelöst und dreimal täglich verabreicht.

### Beispiel 6

Eine im Eiweiß adaptierte Säuglingsmilchnahrung (*Aptamil*® von Milupa) mit 11,8 g Eiweiß, 56,9 g Kohlenhydrate24,9 g Fett, 2,5 g Mineralstoffen, Vitaminen und 45 mg Taurin wird auf übliche Weise in Form eines Perlates hergestellt, das mit 100 mg bis 1000 mg Cycloglycan pro 100 g Säuglingsmilchnahrung vermengt wird.

## Patentansprüche

1. Verwendung von Cycloglycanen mit einer ringförmigen Grundstruktur aus 2 bis 40 Monosacchariden im Ring, der unsubstituiert ist oder der an den diesen Ring bildenden Monosacchariden durch eine oder mehrere funktionelle Gruppe derivatisiert sein kann, wobei diese Cycloglycane auch an einen inerten Träger gebunden oder daran immobilisiert sein können,
zur Herstellung eines diätetischen Mittels oder pharmazeutischen Mittels zur Verringerung oder Verhinderung der Invasion und Infektion von Säugerzellen durch invasive gram-positive und gram-negative, pathogene Bakterien und zur Bekämpfung von durch derartige Bakterien verursachten Erkrankungen.

2. Verwendung nach Anspruch 1,
**dadurch gekennzeichnet,**
**dass** es sich um homopolymere Cycloglycane handelt und/oder der Ring der Cycloglycane aus 6 bis 40 und insbesondere 6 bis 20 Monosacchariden aufgebaut ist.

3. Verwendung nach Anspruch 1 oder 2,
**dadurch gekennzeichnet,**
**dass** der Ring der Cycloglycane aufgebaut ist aus D-Fructose, D-Mannose, L-Fucose, D-N-Acetylglucosamin, D-N-Acetylgalactosamin, D-Xylose, Sialinsäuren, L-Rhamnose, D-Arabinose, D-Allose, D-Talose, L-Idose, D-Ribose, D-Galacturonsäure, Altrose, D-Galactose und Glucosen.

4. Verwendung nach einem der vorhergehenden Ansprüche,
**dadurch gekennzeichnet,**
**dass** eine oder beide der folgenden Merkmale erfüllt sind:
i) eine oder mehrere der OH-Gruppen eines oder mehrerer der den Ring bildenden Monosaccharide ist bzw. sind durch eine NH₂-, SH-, Phosphat- Sulfat-, Nitrat-, Alkyl-, Hydroxyalkyl-, oder Carboxyalkyl-Gruppe ersetzt;
ii) eine oder mehrere der OH-Gruppen sowie der eventuell vorhandenen NH₂- und SH-Gruppen der den Ring bildenden Monosaccharide sind in Form von Ethern, Estern, Amiden und Iminen derivatisiert.

5. Verwendung nach einem der vorhergehenden Ansprüche,
**dadurch gekennzeichnet,**
**dass** die Bindung der Monosaccharide im Ring α-glycosidisch oder β-glycosidisch ist, wobei es sich bei den β-glycosidisch verknüpften Monosacchariden insbesondere um periplasmatische Glycane handelt.

6. Verwendung nach einem der vorhergehenden Ansprüche,
**dadurch gekennzeichnet,**
**dass** die Cycloglycane 6, 7 oder 8 Monosaccharide und insbesondere Glucose-Einheiten im Ring aufweisen.

7. Verwendung nach Anspruch 1,
**dadurch gekennzeichnet,**
**dass** es sich bei den Cycloglycanen um folgende handelt:
α-Cyclodextrin, β-Cyclodextrin; γ-Cyclodextrin;, Cyclo-fructine, Cyclomannine, Cyclogalactine und Cycloaltrine, die auf die im Anspruch 4 beschriebene Weise derivatisiert sein können.

8. Verwendung nach Anspruch 7,
**dadurch gekennzeichnet,**
**dass** es sich bei den derivatisierten Cycloglycanen um Hydroxypropyl-Cyclodextrine handelt.

9. Verwendung nach einem der vorhergehenden Ansprüche,
**dadurch gekennzeichnet,**
**dass** es sich bei dem Träger um ein Peptid, ein Protein, ein Lipid, ein Lipoid, ein Polymer oder ein Biopolymer handelt.

10. Verwendung nach einem der vorhergehenden Ansprüche,
**dadurch gekennzeichnet,**
**dass** das Mittel zur oralen, lingualen, nasalen, bronchialen, vaginalen, topischen (Haut und Schleimhaut) oder per os Verabreichung, zur Verabreichung mittels einer Sonde in den Magen eines Menschen oder eines Tieres oder zur Verabreichung als Infusion dient.

11. Verwendung nach einem der vorhergehenden Ansprüche,
**dadurch gekennzeichnet,**
**dass** die Cycloglycane in einer Menge von mindestens 1 mg pro kg Körpergewicht und Tag einem Menschen oder einem Tier verabreicht werden.

12. Verwendung nach einem der vorhergehenden Ansprüche,
zur Prävention und Behandlung von Infektionen des Gastrointestinaltraktes, des Blutsystems, der Atemwege, des Urogenitaltraktes sowie des Nasen-Rachenraumes, insbesondere bei Listeriosen.

13. Verwendung nach einem der vorhergehenden Ansprüche,
**dadurch gekennzeichnet,**
**dass** es sich bei den Bakterien um Listerien handelt.

14. Verwendung nach einem der vorhergehenden Ansprüche,
**dadurch gekennzeichnet,**
**dass** zur Herstellung des Mittels ein weiteres Kohlenhydrat oder mehrere weitere Kohlenhydrate, bei denen es sich nicht um ein im Anspruch 1 beschriebenes Cycloglycan handelt, ein weiterer Wirkstoff oder mehrere weitere Wirkstoffe, und/oder ein weiteren Inhaltsstoff, der für das entsprechende Mittel bekannt und geeignet ist, oder mehrere derartige Inhaltsstoffe eingesetzt werden, wobei im Falle eines pharmazeutischen Mittels zusätzlich ein üblicher Hilfsstoff oder mehrere übliche Hilfsstoffe, wozu Verdünnungsmittel, feucht-haltende Mitteln, Verdickungsmittel, Geschmacksstoffe, Süßungsmittel und Träger zählen, verwendet werden kann.

15. Cycloglycane mit einer ringförmigen Grundstruktur aus 2 bis 40 Monosacchariden im Ring, der unsubstituiert ist oder der an den diesen Ring bildenden Monosacchariden durch eine oder mehrere funktionelle Gruppe derivatisiert sein kann, wobei diese Cycloglycane auch an einen inerten Träger gebunden oder daran immobilisiert sein können,
zur Verwendung bei der Verringerung oder Verhinderung der Invasion und Infektion von Säugerzellen durch invasive gram-positive und gram-negative, pathogene Bakterien und zur Bekämpfung von durch derartige Bakterien verursachten Erkrankungen.

16. Cycloglycane nach Anspruch 15,
**dadurch gekennzeichnet, dass**
es sich um solche nach mindestens einem der in den Ansprüche 2 bis 9 genannten handelt.

## Claims

1. Use of cycloglycans having a ring-shaped base structure of 2 to 40 monosaccharides in the ring, which is unsubstituted or may be derivatized at the monosaccharides forming said ring, by one or more functional group(s), wherein said cycloglycans may also be bound to an inert carrier or may be immobilized thereon,
for preparing an dietetic composition or a pharmaceutical composition for reducing or preventing the invasion and infection of mammalian cells by invasive gram-positive and gram-negative pathogenic bacteria, and for combating diseases caused by such bacteria.

2. Use according to claim 1,
**characterized in that**
the cycloglycans are homopolymeric cycloglycans and/or the ring of the cycloglycans is made up of 6 to 40, and in particular 6 to 20 monosaccharides.

3. Use according to claim 1 or 2,
**characterized in that**
the ring of the cycloglycans is made up of D-fructose, D-mannose, L-fucose, D-N-acetyl glucosamine, D-N-acetyl galactosamine, D-xylose, sialic acids, L-rhamnose, D-arabinose, D-allose, D-talose, L-idose, D-ribose, D-galacturonic acid, altrose, D-galactose and glucoses.

4. Use according to any one of the preceding claims,
**characterized in that**
one or both of the following criteria are met:
i) one or more of the OH groups of one or more of the monosaccharides forming the ring is or are substituted by an NH₂ group, SH group, phosphate group, sulfate group, nitrate group, alkyl group, hydroxyalkyl group or carboxyalkyl group.
ii) one or more of the OH groups as well as of the - if present - NH₂ and SH groups of the monosaccharides forming the ring are derivatized in the form of ethers, esters, amides and imines.

5. Use according to any one of the preceding claims,
**characterized in that**
the linkage of the monosaccharides in the ring is α-glycosidic or β-glycosidic, with the β-glycosidically linked monosaccharides in particular being periplasmic glycans.

6. Use according to any one of the preceding claims,
**characterized in that**
the cycloglycans have 6, 7 or 8 monosaccharides and in particular glucose units in the ring.

7. Use according to claim 1,
**characterized in that**
the cycloglycans are the following:
α-cyclodextrin, β-cyclodextrin, γ-cyclodextrin; cyclofructines, cyclomannines, cyclogalactines and cycloaltrines, which may be derivatized in the manner described in claim 4.

8. Use according to claim 7,
**characterized in that**
the derivatized cycloglycans are hydroxypropyl cyclodextrins.

9. Use according to any one of the preceding claims,
**characterized in that**
the carrier is a peptide, a protein, a lipid, a lipoid, a polymer or a biopolymer.

10. Use according to claim 10,
**characterized in that**
the composition serves for an oral, lingual, nasal, bronchial, vaginal, topical (skin and mucosa) and *per os* administration, for an administration by means of a probe into the stomach of a human or an animal, or for an administration as an infusion.

11. Use according to any one of the preceding claims,
**characterized in that**
the cycloglycans are administered once daily in an amount of at least 1 mg per kg of body weight to a human or an animal.

12. Use according to any one of the preceding claims,
for the prevention and treatment of infections of the gastrointestinal tract, blood system, respiratory passages, urogenital tract, as well as the nasopharynx, in particular in case of listerioses.

13. Use according to any one of the preceding claims,
**characterized in that**
the bacteria are listeria.

14. Composition according to any one of the preceding claims,
**characterized in that**
it may contain a further carbohydrate or more further carbohydrates, which are not a cycloglycan as described in claim 1, a further active agent or several further active agents and/or a further ingredient, which is known and suited for the corresponding composition, or more of such ingredients, wherein in the case of a pharmaceutical composition a usual auxiliary agent or several usual auxiliary agents, including diluents, moisturizing agents, thickening agents, flavoring agents, sweetening agents and carriers, may be present.

15. Cycloglycans having a ring-shaped base structure of 2 to 40 monosaccharides in the ring, which is unsubstituted or may be derivatized at the monosaccharides forming said ring by one or more functional group(s), wherein said cycloglycans may also be bound to an inert carrier or immobilized thereon,
for the use in the reduction or prevention of the invasion and infection of mammalian cells by invasive gram-positive and gram-negative pathogenic bacteria, and for combating diseases caused by such bacteria.

16. Cycloglycans according to claim 15,
**characterized in that**
they are those as described in at least one of the claims 2 to 9.

## Revendications

1. Utilisation de cycloglycanes présentant une structure de base cyclique composée de 2 à 40 monosaccharides dans le cycle, lequel cycle est non substitué ou dérivé au niveau des monosaccharides formant ce cycle par un ou plusieurs groupes fonctionnels, ces cycloglycanes pouvant également être liés à un substrat inerte ou immobilisés sur celui-ci,
pour la préparation d'un produit diététique ou d'un agent pharmaceutique destiné à réduire ou empêcher l'invasion et l'infection de cellules de mammifère par des bactéries pathogènes invasives Gram-positives et Gram-négatives, et pour la lutte contre les maladies provoquées par les bactéries de ce type.

2. Utilisation selon la revendication 1,
**caractérisée en ce que**
il s'agit de cycloglycanes homopolymères et/ou le cycle des cycloglycanes est constitué de 6 à 40 et en particulier de 6 à 20 monosaccharides.

3. Utilisation selon la revendication 1 ou 2,
**caractérisée en ce que**
le cycle des cycloglycanes est constitué de D-fructose, de D-mannose, de L-fucose, de D-N-acétylglucosamine, de D-N-acétylgalactosamine, de D-xylose, d'acides sialiques, de L-rhamnose, de D-arabinose, de D-allose, de D-talose, de L-idose, de D-ribose, d'acide D-galacturonique, d'altrose, de D-galactose et de glucoses.

4. Utilisation selon l'une quelconque des revendications précédentes,
**caractérisée en ce que**
l'une ou les deux conditions suivantes sont remplies :
i) un ou plusieurs des groupes OH d'un ou de plusieurs des monosaccharides formant le cycle sont substitués par un groupe NH₂, SH, phosphate, sulfate, nitrate, alkyle, hydroxyalkyle ou carboxyalkyle ;
ii) un ou plusieurs des groupes OH ainsi que des groupes NH₂ et SH éventuellement présents des monosaccharides formant le cycle sont dérivés sous forme d'éthers, d'esters, d'amides et d'imines.

5. Utilisation selon l'une quelconque des revendications précédentes,
**caractérisée en ce que**
la liaison des monosaccharides dans le cycle est de type α-glycosidique ou β-glycosidique, les monosaccharides à liaison β-glycosidique étant en particulier des glycanes périplasmiques.

6. Utilisation selon l'une quelconque des revendications précédentes,
**caractérisée en ce que**
les cycloglycanes présentent 6, 7 ou 8 monosaccharides et en particulier des motifs glucose dans le cycle.

7. Utilisation selon la revendication 1,
**caractérisée en ce que**
il s'agit des cycloglycanes suivants : α-cyclodextrine, β-cyclodextrine, γ-cyclodextrine, cyclofructines, cyclomannines, cyclogalactines et cycloaltrines, qui peuvent être dérivés selon la manière décrite dans la revendication 4.

8. Utilisation selon la revendication 7,
**caractérisée en ce que**
les cycloglycanes dérivés sont des hydroxypropylcyclodextrines.

9. Utilisation selon l'une quelconque des revendications précédentes,
**caractérisée en ce que**
le substrat est un peptide, une protéine, un lipide, un lipoïde, un polymère ou un biopolymère.

10. Utilisation selon l'une quelconque des revendications précédentes,
**caractérisée en ce que**
l'agent est destiné à une administration par voie orale, linguale, nasale, bronchique, vaginale, topique (peau et muqueuses) ou *per os*, à une administration au moyen d'une sonde gastrique chez un être humain ou chez un animal ou il est destiné à une administration sous forme de perfusion.

11. Utilisation selon l'une quelconque des revendications précédentes,
**caractérisée en ce que**
les cycloglycanes sont administrés à un être humain ou à un animal à raison d'au moins 1 mg par kg de poids corporel et par jour.

12. Utilisation selon l'une quelconque des revendications précédentes,
pour la prévention et le traitement d'infections du tractus gastro-intestinal, du système sanguin, des voies respiratoires, du tractus uro-génital ainsi que de la cavité bucco-nasale, en particulier dans le cas de listérioses.

13. Utilisation selon l'une quelconque des revendications précédentes,
**caractérisée en ce que**
les bactéries sont des *Listeria.*

14. Utilisation selon l'une quelconque des revendications précédentes,
**caractérisée en ce que**
un ou plusieurs autres glucides qui ne sont pas un cycloglycane tel que décrit dans la revendication 1, un ou plusieurs autres principes actifs et/ou un autre composant connu et approprié pour l'agent correspondant, ou plusieurs des composants de ce type, sont mis en oeuvre pour la préparation de l'agent, dans laquelle, dans le cas d'un agent pharmaceutique, un ou plusieurs adjuvants classiques supplémentaires, parmi lesquels on compte les diluants, les agents mouillants, les épaississants, les aromatisants, les édulcorants et les charges, peuvent être utilisés.

15. Cycloglycanes présentant une structure de base cyclique composée de 2 à 40 monosaccharides dans le cycle, lequel cycle est non substitué ou dérivé au niveau des monosaccharides formant ce cycle par un ou plusieurs groupes fonctionnels, ces cycloglycanes pouvant également être liés à un substrat inerte ou immobilisés sur celui-ci,
pour une utilisation destinée à réduire ou à empêcher l'invasion et l'infection de cellules de mammifère par des bactéries pathogènes invasives Gram-positives et Gram-négatives, et à lutter contre les maladies provoquées par les bactéries de ce type.

16. Cycloglycanes selon la revendication 15,
**caractérisés en ce que**
il s'agit des cycloglycanes mentionnés dans au moins l'une quelconque des revendications 2 à 9.
